(19) **Europäisches Patentamt / European Patent Office / Office européen des brevets**

(11) **EP 3 137 050 B1**

(12) **FASCICULE DE BREVET EUROPEEN**

(45) Date de publication et mention de la délivrance du brevet:
**04.03.2020 Bulletin 2020/10**

(21) Numéro de dépôt: **15718939.0**

(22) Date de dépôt: **30.04.2015**

(51) Int Cl.:
*A61K 8/73* (2006.01)     *A61K 31/715* (2006.01)
*A61K 31/727* (2006.01)   *A61K 31/728* (2006.01)
*A61K 31/737* (2006.01)   *A61K 8/33* (2006.01)
*A61K 8/67* (2006.01)     *A61Q 19/08* (2006.01)
*A61K 9/00* (2006.01)     *A61K 45/06* (2006.01)
*A61K 31/11* (2006.01)    *A61K 31/7048* (2006.01)

(86) Numéro de dépôt international:
**PCT/EP2015/059544**

(87) Numéro de publication internationale:
**WO 2015/166063 (05.11.2015 Gazette 2015/44)**

(54) **ASSOCIATION D'UN ACIDE HYALURONIQUE ET D'UN POLYSACCHARIDE SULFATE**

KOMBINATION EINER HYALURONSÄURE UND EINES SULFATIERTEN POLYSACCHARIDS

COMBINATION OF A HYALURONIC ACID AND OF A SULPHATED POLYSACCHARIDE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.04.2014 FR 1453973**

(43) Date de publication de la demande:
**08.03.2017 Bulletin 2017/10**

(73) Titulaire: **Pierre Fabre Dermo-Cosmétique**
**92100 Boulogne-Billancourt (FR)**

(72) Inventeurs:
• **POIGNY, Stéphane**
**F-31600 Saubens (FR)**
• **HERNANDEZ-PIGEON, Hélène**
**F-31270 Cugnaux (FR)**
• **SAURAT, Jean-Hilaire**
**CH-1206 Geneve (CH)**
• **KAYA, Gürkan**
**CH-1206 Geneve (CH)**

(74) Mandataire: **Regimbeau**
**20, rue de Chazelles**
**75847 Paris Cedex 17 (FR)**

(56) Documents cités:
WO-A1-2008/068297    WO-A1-2008/071245
WO-A1-2013/017807    WO-A1-2013/150253
US-A1- 2009 286 756    US-B1- 6 572 868

EP 3 137 050 B1

**Description**

DOMAINE DE L'INVENTION

La présente invention concerne une nouvelle association d'un acide hyaluronique ou un de ses sels et d'un polysaccharide sulfaté de bas poids moléculaire ; ainsi que l'utilisation de ladite association dans les domaines de l'anti-âge et de la cicatrisation.

ART ANTERIEUR

L'acide hyaluronique (AH) est une molécule au rôle majeur dans la peau. C'est en effet la composante principale de la matrice extracellulaire. Cette dernière désigne l'ensemble de macromolécules extracellulaires du tissu conjonctif. Elle est constituée en grande partie de glycoprotéines, de protéines pures ainsi que des glycosaminoglycanes. L'AH est un glycosaminoglycane linéaire non sulfaté composé d'unités répétitives de disaccharides eux-mêmes composés de D-acide glucuronique et N-acétyl-D-glucosamine liés par des liaisons glycosidiques alternées β1-3 et β1-4 entre les dimères (Tammi R., Agren U.M., Tuhkanen A.L., Tammi M. Hyaluronan metabolism in skin. Progress in Histochemistry & Cytochemistry 29(2):1-81, 1994). Sous sa forme native, l'AH est un polymère de très haut poids moléculaire compris entre 600 000 Da et 3 MDa (Toole BP. Hyaluronan: from extracellular glue to peracellular cue. Nat Rev Cancer 2004, 4:538-539).

Avec l'âge, la quantité d'AH et son degré de polymérisation diminuent, résultant en une moindre quantité d'eau retenue dans la matrice extracellulaire. La peau subit alors un processus de vieillissement qui aboutit à une augmentation de la fibrose et à une baisse de la teneur en fibres élastiques. Au cours du processus de vieillissement, on observe une modification de la structure et des fonctions cutanées. Ce vieillissement est de nature physiologique mais peut être également photo-induit, c'est-à-dire dû à une exposition répétée de la peau à la lumière solaire, notamment ultraviolette. Les principaux signes cliniques du vieillissement cutané sont l'apparition de ridules et de rides profondes, qui augmentent bien évidemment avec l'âge. Les sillons et les rides se marquent ; la peau se creuse et perd sa fermeté ; en surface, la peau perd sa luminosité.

Les polysaccharides sulfatés comprennent entre autres les fucanes sulfatés et les ulvanes sulfatés de poids moléculaire compris entre 5 et 25kDa.

Ces polysaccharides sulfatés de bas poids moléculaires ont des propriétés biologiques intéressantes : inhibition de la dégradation du collagène et de l'élastine, restructuration, action anti-inflammatoire, et induction de la production d'AH.

Au cours du vieillissement, les fibroblastes entrent en sénescence et leur capacité de prolifération diminue fortement. Ainsi, le fait de pouvoir stimuler à nouveau la prolifération de ces cellules est une des voies étudiées pour lutter contre le vieillissement cutané.

Par ailleurs, la cicatrisation des plaies est un processus biologique complexe et dynamique qui met en jeu l'interaction de nombreux facteurs locaux et systémiques dans la réparation normale des tissus. La progression de la cicatrisation comporte trois phases interdépendantes : l'hémostase et l'inflammation, la prolifération et le remodelage (General principles of wound healing. Witte MB, Barbul A. Surg Clin North Am. 1997 Jun; 77(3):509-28.). La prolifération implique trois processus bien observables : la granulation, la contraction et la réépithélialisation.

Au cours de la granulation, on observe la prolifération et la migration vers le lit de la plaie des cellules qui interviendront dans le reste du processus de réparation. Ainsi, on y retrouve des macrophages, des fibroblastes et des cellules endothéliales. Les macrophages libèrent constamment des facteurs chimiotactiques et des facteurs de croissance. Les fibroblastes construisent la nouvelle matrice cellulaire nécessaire à la croissance des cellules au fond de la plaie. Cet échafaudage favorise la migration cellulaire. Enfin, les cellules endothéliales déclenchent la formation de bourgeons vasculaires qui constitueront de nouveaux capillaires, ce qui permettra de rétablir la perfusion et d'assurer l'apport en oxygène et en nutriments essentiels à l'activité métabolique des cellules dans la plaie.

La contraction de la plaie est un mécanisme de réduction de la taille de la plaie et les fibroblastes jouent un rôle de premier plan dans cette contraction.

La ré-épithélialisation consiste en la régénération d'un épiderme qui recouvre une plaie pour reformer une barrière efficace contre l'environnement extérieur, capable de se pigmenter et de retrouver ses fonctions sensorielles et immunitaires. Elle implique donc les processus cellulaires de migration et de prolifération des kératinocytes, mais aussi la différenciation de ce néo-épithélium et la restauration d'une membrane basale qui reconnecte le derme et l'épiderme. Lorsque la migration des cellules basales en direction du centre de la plaie permet aux deux bords de la plaie de se rejoindre, une vague de mitose cellulaire se produit pour combler les espaces laissés par la migration et fournir des cellules pour le tissu épithélial en régénération tri-dimensionnelle.

Les étapes de prolifération des cellules kératinocytaires, de fibroblastes ou de cellules endothéliales peuvent être considérées comme un des phénomènes fonctionnels témoignant de l'activité cicatrisante d'un actif. Une augmentation de la prolifération des fibroblastes participerait à la cicatrisation d'une plaie profonde (atteinte du derme), alors que l'augmentation de la prolifération des kératinocytes participerait à la ré-épithélialisation.

Il existe toujours un besoin de proposer de nouvelles compositions cosmétiques pour lutter contre les signes du vieillissement cutané.

La demanderesse a mis en évidence l'existence d'une synergie entre l'acide hyaluronique et un polysaccharide sulfaté de bas poids moléculaire sur la prolifération des fibroblastes. Cette activité est particulièrement intéressante dans le domaine de l'anti-âge mais aussi dans la régénération tissulaire et la cicatrisation des lésions cutanées.

DESCRIPTION DETAILLEE

La présente invention a pour objet une association comprenant un acide hyaluronique ou un de ses sels et un polysaccharide sulfaté dont le poids moléculaire est compris entre 5 et 25 kDa et qui est un fucane sulfaté.

Dans le cadre de la présente invention, les termes « acide hyaluronique », « fragments d'acide hyaluronique », « AH » et « hyaluronane » sont utilisés indifféremment pour désigner de l'acide hyaluronique. Lorsque l'acide hyaluronique est sous forme de sel, on parlera de hyaluronate.

Dans un mode de réalisation particulier de l'invention, l'association comprend un acide hyaluronique ou un de ses sels dont la masse moléculaire moyenne en poids (Mw) sera comprise entre 50 000 et 750 000 Da.

Dans un mode de réalisation particulier de l'invention, le fragment d'AH ou un de ses sels se caractérise par une masse moléculaire moyenne en poids comprise entre 60 et 120 kDa, ladite masse moléculaire étant mesurée par une méthode analytique combinant une technique de chromatographie d'exclusion de taille (Size Exclusion Chromatography - SEC) avec un détecteur à diffusion laser multi-angle (Multi-Angle Light Scattering photometer - MALS) couplé à un viscosimètre (VIS) et à un réfractomètre différentiel (RI).

Dans un mode de réalisation particulier, il s'agira du hyaluronate de sodium.

La masse moléculaire de l'AH ou un de ses sels peut être mesurée à l'aide de la méthode de la pharmacopée européenne qui mesure la viscosité intrinsèque avec un viscosimètre capillaire de Ubbelohde (cf. Pharmacopée européenne 7.6, monographie du hyaluronate de sodium réf. : 01/2011 :1472). Cette valeur de viscosité est ensuite reliée à la masse moléculaire moyenne par la relation de Mark-Houwink. Cette méthode est longue et nécessite une parfaite reproductibilité.

Dans le cadre de la présente invention, la masse moléculaire moyenne en poids de l'AH ou un de ses sels est mesurée par la méthode SEC-MALS-VIS-RI qui est une méthode analytique combinant une technique de chromatographie d'exclusion de taille (Size Exclusion Chromatography - SEC) avec un détecteur à diffusion laser multi-angle (Multi-Angle Light scattering photometer - MALS) couplé à un viscosimètre (VIS) et un réfractomètre différentiel. Cette technique permet d'obtenir la masse moléculaire moyenne en poids (Mw).

Cette technique permet de caractériser les masses moléculaires des AH de façon précise et reproductible (Stepan Podzimek & al. Solution of Hyaluronic Acid and Comparaison of SEC-MALLS-VIS Data with Off-line Capillary Viscometry Journal of Applied Polymer science 2009).

Le couplage d'une chromatographie d'exclusion de taille SEC à un détecteur MALS permet après injection d'une solution de polymères dans un système chromatographique, de séparer ces polymères par taille dans la colonne chromatographique, de mesurer cette taille par diffusion de lumière et de les quantifier à l'aide d'un réfractomètre différentiel ou d'un spectromètre UV.

- L'acide hyaluronique ou un de ses sels est solubilisé dans une solution aqueuse de NaCl 0,1 M puis élué sur une colonne remplie de billes de polystyrène-divinyl-benzène de porosité calibrée. Les grosses chaînes polymériques ne passent pas dans tous les pores et sont donc éluées avant les petites chaînes.
- Le détecteur MALS mesure la diffusion de la lumière incidente à différents angles. Ces angles permettent, par extrapolation, de mesurer R0 qui est la diffusion à angle 0. R0 est directement proportionnel à la taille de la molécule.
- La réponse $Si$ du réfractomètre différentiel est proportionnelle à la masse totale $Ci$ du polymère de degré de polymérisation $i$ suivant l'équation (1) suivante :

$$\textbf{Si = K'.dn/dc.Ci}$$

où : $Si$ est donc la réponse du réfractomètre différentiel,
K' est une constante liée à l'appareil, et
$C_i$ est la masse (poids) totale du polymère de degré de polymérisation $i$ ($Ci = Ni \times Mi$), $N_i$ étant le nombre de chaînes de masse molaire $M_i$.

L'incrément d'indice de réfraction dn/dc est en outre une valeur spécifique du polymère étudié.

Ce rapport dn/dc peut être mesuré selon le protocole suivant exemplifié avec le hyaluronate de sodium.

a/ Appareil et préparation des solutions :

Les mesures sont effectuées sur un réfractomètre tel que le modèle Brice-Phoenix ayant comme source de lumière incidente un laser hélium-néon ($\lambda$=633nm).

Différentes concentrations en polymère à étudier sont préparées indépendamment par pesée (durée de mise en solution = 24 heures). Les indices de réfractions (dn) respectifs sont ensuite mesurés à l'aide du réfractomètre.

b/ Résultat

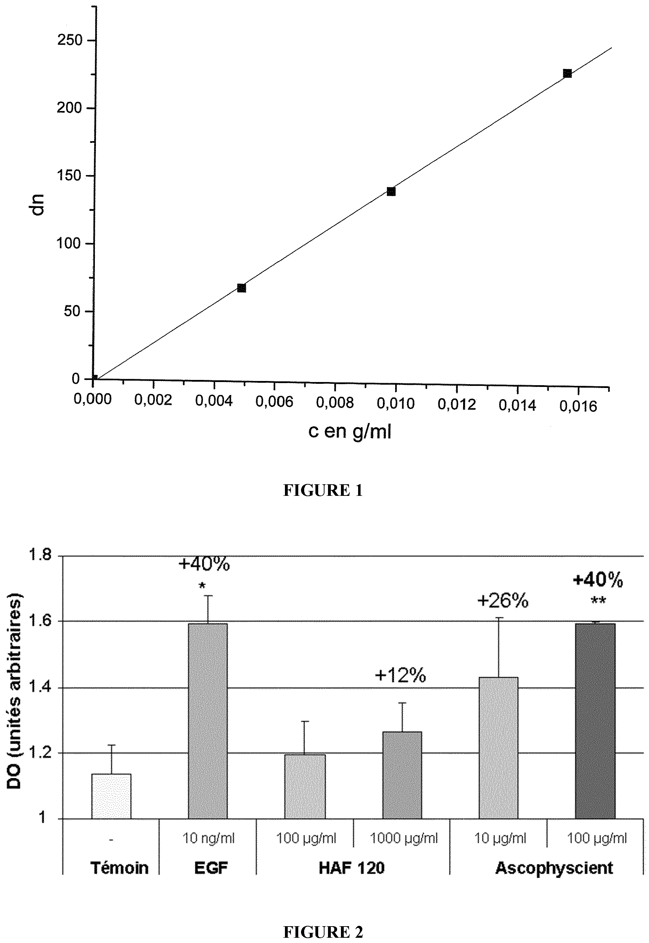

Les résultats sont présentés sous forme de graphique dn = f(concentration) (voir Figure 1). Une régression linéaire du type Y = A + B*X est alors déterminée.

Dans le cas du hyaluronate de sodium, les valeurs A et B déterminées sont respectivement A = -2,030 et B = 14927,911.

Le rapport dn/dc correspond alors à k x B où k représente la constante de l'appareil qui est égale à $0,97.10^{-5}$ dans le présent exemple.

Pour le hyaluronate de sodium, dn/dc est donc évalué à $0,97.10^{-5}$ x 14927,911 = 0,145 ml/g.

La formule suivante donne la relation entre tous les paramètres mesurés :

$$R0 = K.Ci.Mi$$

où : R0 est le rapport de RAYLEIG à angle de diffusion 0,

K est la constante de l'appareil,

$Ci$ représente la masse totale du polymère de degré de polymérisation $i$ [calculée suivant l'équation (1)] de la solution injectée,

et $Mi$ représente la masse molaire de la chaîne polymérique recherchée.

La masse moléculaire moyenne en poids Mw peut alors être calculée selon la formule :

$$Mw = \Sigma CiMi / \Sigma Ci.$$

Toutes les masses moléculaires sont exprimées en Daltons.

L'acide hyaluronique ou son sel, objet de la présente invention, peut être obtenu selon un des procédés connus de l'homme du métier, tels que ceux rappelés dans le document EP1987153.

L'AH est principalement obtenu, de façon industrielle, par fermentation bactérienne : les filaments d'acide hyaluronique sont synthétisés par des bactéries.

Ainsi dans un mode de réalisation particulier de l'invention, un AH natif de haut poids moléculaire allant généralement de 1 MDa à 2 MDa est obtenu par fermentation bactérienne en utilisant une souche bactérienne sélectionnée.

Le polymère obtenu peut alors être séparé de la bactérie. La solution est ensuite purifiée puis hydrolysée par hydrolyse acide contrôlée jusqu'à l'obtention du poids moléculaire souhaité.

Dans le cas de la préparation du hyaluronate de sodium, on procédera à une étape de neutralisation par ajout de NaOH.

Le sel de l'acide hyaluronique sera préférentiellement le hyaluronate de sodium.

Dans le cadre de la présente invention, les polysaccharides sulfatés de bas poids moléculaire sont des fucanes sulfatés de poids moléculaire compris entre 5 et 25kDa.

Les fucanes sulfatés sont des polysaccharides comprenant du L-fucose sulfaté. Ces polysaccharides peuvent être extraits notamment d'algues brunes, par exemple de l'ordre des Fucales ou des Laminariales.

Selon un mode de réalisation particulier, le fucane sulfaté de bas poids moléculaire est tel que décrit dans WO 2010/086197.

On peut citer la spécialité commerciale Ascophyscient® d'Algues et Mer extrait de l'algue *Ascophyllum nodosum*.

Selon un mode de réalisation particulier de l'invention, le ratio massique acide hyaluronique / polysaccharide est compris entre 0,1 et 10.

Selon un autre mode de réalisation particulier de l'invention, le ratio massique acide hyaluronique / polysaccharide est compris entre 0,5 et 5, et de préférence entre 0,5 et 2.

La présente invention porte, en outre, sur une association d'acide hyaluronique ou un de ses sels et de polysaccharide sulfaté de bas poids moléculaire selon l'invention (c'est-à-dire de poids moléculaire compris entre 5 et 25 kDa) et qui est un fucane sulfaté pour son utilisation pour favoriser la prolifération des fibroblastes.

La présente invention concerne également une composition cosmétique comprenant, en tant que principe actif anti-âge, une association d'un acide hyaluronique ou un de ses sels et d'un polysaccharide sulfaté de poids moléculaire compris entre 5 et 25 kDa et qui est un fucane sulfaté et comprenant en outre au moins un excipient cosmétiquement acceptable.

De préférence, les excipients cosmétiquement acceptables sont adaptés à une administration topique.

Les excipients acceptables permettent en particulier d'assurer une bonne stabilité, une texture et un toucher agréables. Il peut aussi s'agir par exemple d'agents de formulations ou d'additifs d'usage connu et classique en cosmétique : on peut citer des tensioactifs, colorants, conservateurs, parfums, agents filmogènes, épaississants, etc.

Les compositions anti-âge peuvent se présenter sous les formes qui sont habituellement connues pour une administration topique sur la peau, c'est-à-dire notamment des crèmes, émulsions, lotions, sérums, masques, combleurs de rides, contours des yeux, ...

L'objet de la présente invention vise l'utilisation cosmétique de l'association selon la présente invention ou de cette composition cosmétique selon l'invention pour lutter contre les signes du vieillissement cutané. L'utilisation cosmétique de l'association selon la présente invention ou la composition cosmétique selon l'invention est plus particulièrement destinée à redonner de la matière à la peau, renforcer sa fermeté et réduire visiblement les rides marquées et les sillons profonds.

La présente invention concerne également une méthode pour lutter contre les signes du vieillissement cutané comprenant l'administration, de préférence topique, d'une quantité efficace d'une association selon l'invention ou d'une composition cosmétique selon l'invention à une personne en ayant besoin.

Un autre objet de la présente invention concerne une composition dermatologique destinée à accélérer la réparation cutanée afin de rétablir l'intégrité et la qualité de la peau comprenant en tant que principe actif dermatologique ou cosmétique l'association d'un acide hyaluronique ou un de ses sels et un polysaccharide sulfaté dont le poids moléculaire est compris entre 5 et 25 kDa et qui est un fucane sulfaté ci-dessus mentionné et comprenant en outre au moins un excipient dermatologiquement ou cosmétiquement acceptable.

Dans un mode de réalisation préféré de l'invention, la composition est destinée à une application topique.

Les excipients dermatologiquement (pharmaceutiquement) ou cosmétiquement compatibles peuvent être tout excipient parmi ceux connus de l'homme de l'art en vue d'obtenir une composition pour l'application topique sous forme d'un lait, d'une crème, d'un baume, d'une huile, d'une lotion, d'un gel, d'un gel moussant, d'une pommade, d'un spray, etc.

Dans un mode de réalisation préféré, la composition sera sous forme d'une crème ou d'une pommade.

Dans un mode de réalisation particulier, les compositions dermatologiques et cosmétiques selon l'invention comprennent au moins un autre principe actif.

Cet autre principe actif pourra notamment être sélectionné dans le groupe comprenant des agents anti-âge, cicatrisants, apaisants, antiprurit, antioxydants, anti-radicalaires, anti-UV, stimulant la synthèse de macromolécules dermiques ou le métabolisme énergétique, hydratants, dépigmentants, antibactériens, antifongiques, anti-inflammatoires, ou encore anesthésiques.

Selon un mode de réalisation particulier, les compositions selon l'invention, de préférence cosmétiques, comprennent en outre du rétinaldéhyde. Cet actif anti-âge complémentaire est un précurseur direct d'acide rétinoïque pour un effet réactivateur du métabolisme cellulaire immédiat. Le rétinaldéhyde augmente l'expression des récepteurs de l'AH, les CD44 et induit la synthèse d'AH.

La composition selon l'invention comprend alors une association d'acide hyaluronique ou un de ses sels, de polysaccharide sulfaté de bas poids moléculaire qui est un fucane sulfaté et de rétinaldéhyde. Une telle association a un effet restructurant et redensifiant dans la jonction dermo-épidermique et le derme papillaire.

Selon un autre mode de réalisation de l'invention, la composition comprendra en outre un dérivé de tocophérol dont le delta tocophéryl glucopyranoside : ses effets antioxydants puissants protègent la peau et préservent sa luminosité.

La composition selon l'invention comprend alors une association d'acide hyaluronique ou un de ses sels, de polysaccharide sulfaté de bas poids moléculaire qui est un fucane sulfaté et de delta tocophéryl glucopyranoside.

Selon un autre mode de réalisation particulier, l'autre principe actif sera choisi parmi les agents cicatrisants, les agents apaisants et leurs mélanges. Un tel principe actif sera utilisé de préférence dans des compositions dermatologiques.

Enfin, un autre objet de la présente invention vise une association d'acide hyaluronique ou un de ses sels et de polysaccharide sulfaté de bas poids moléculaire qui est un fucane sulfaté ou une composition dermatologique la contenant selon l'invention pour son utilisation comme médicament (plus particulièrement dermatologique), destiné notamment au traitement et à la cicatrisation des lésions cutanées.

La présente invention concerne également l'utilisation d'une association d'un acide hyaluronique ou un de ses sels et d'un polysaccharide sulfaté de poids moléculaire compris entre 5 et 25 kDa et qui est un fucane sulfaté pour la préparation d'un médicament (plus particulièrement dermatologique), destiné notamment au traitement et à la cicatrisation des lésions cutanées.

La présente invention concerne également une méthode pour traiter et cicatriser des lésions cutanées comprenant l'administration, de préférence topique, d'une quantité efficace d'une association d'un acide hyaluronique ou un de ses sels et d'un polysaccharide sulfaté de poids moléculaire compris entre 5 et 25 kDa et qui est un fucane sulfaté ou une composition dermatologique la contenant selon l'invention à une personne en ayant besoin.

Les compositions dermatologiques et cosmétiques selon l'invention sont destinées notamment aux soins des peaux lésées :

- suite à des actes / traitements invasifs : actes chirurgicaux (exérèses, shavings) avec ou sans suture, cryothérapie, laser ablatif, peelings moyens ou forts, mésothérapie, curetage,
- en post traumatique lors de coupures ou brûlures superficielles,
- suite à des actes superficiels (non invasifs) nécessitant un produit cicatrisant qui accélère la récupération cutanée, utilisable au long cours (jusqu'à réparation complète de la peau),
- suite à une altération extérieure légère : écorchures superficielles, coup de soleil.

Le traitement des lésions de la peau et des muqueuses selon l'invention pourra notamment comprendre le traitement des coupures, des sutures, des écorchures, des éraflures, des égratignures, des cicatrices post-chirurgie ou post-acte de dermatologie esthétique, des brûlures superficielles, des coups de soleil.

La présente invention porte, en outre, sur l'utilisation d'une composition cosmétique selon l'invention destinée à l'amélioration de la cicatrisation et de la réparation cutanée.

L'invention sera mieux comprise à la lecture des résultats ci-dessous qui l'illustrent sans en limiter la portée.

FIGURES

La Figure 1 représente l'indice de réfraction dn en fonction de la concentration c pour le hyaluronate de sodium.

La Figure 2 représente l'effet d'AH selon l'invention (HAF120) à 100 et à 1000 $\mu$g/ml et d'Ascophyscient® à 10 et à 100 $\mu$g/ml sur la prolifération des fibroblastes. * : $p < 0.05$ et ** : $p < 0.01$. Expérience représentative de 3 expériences indépendantes.

La Figure 3 représente l'effet d'AH selon l'invention (HAF120) à 100 $\mu$g/ml, Ascophyscient® à 10 $\mu$g/ml et leur association sur la prolifération des fibroblastes. * : $p < 0.05$. Expérience représentative de 3 expériences indépendantes.

La Figure 4 représente l'effet d'AH selon l'invention (HAF120) à 1000 $\mu$g/ml, Ascophyscient® à 10 $\mu$g/ml et leur association sur la prolifération des fibroblastes. * : $p < 0.05$ et ** : $p < 0.01$. Expérience représentative de 3 expériences indépendantes.

EXEMPLES

EVALUATION PHARMACOLOGIQUE de l'association d'un fragment de hyaluronate de sodium de masse moléculaire moyenne en poids comprise entre 60 et 120kDa (HAF120) et d'un fucane sulfaté de bas poids moléculaire (5-25kDa) sur la prolifération des fibroblastes

Protocole :

La technique utilisée est celle de l'incorporation d'un nucléotide, la 5-bromo-2'-déoxyuridine (BrdU), analogue de la thymidine, dans l'ADN des cellules en phase S, à 37°C. Cette technique permet de quantifier les cellules dont l'avancée dans le cycle cellulaire est caractéristique d'une cellule proliférative (phase S ou de synthèse de l'ADN).

Les FNH (fibroblastes normaux humains), isolés de déchets opératoires cutanés, sont habituellement cultivés dans du DMEM (Dulbecco's Modified Eagle Médium) avec 10 % de SVF (sérum de veau fœtal). Dans nos conditions expérimentales, les dilutions des produits à tester sont réalisées dans du DMEM avec 3 % de SVF.

Les cellules sont d'abord privées de SVF pendant 24 h pour arrêter la croissance cellulaire avant de les incuber en présence des molécules à évaluer pendant 44 h à 37°C, dans une atmosphère à 5 % de $CO_2$.

L'incorporation du BrdU, proportionnelle au taux de prolifération cellulaire, est évaluée par un système d'anticorps anti-BrdU (Roche Applied Science). L'absorbance correspondante (DO) est mesurée à 450 nm. Cette donnée est donc proportionnelle au taux de prolifération cellulaire.

Les actifs évalués sont les suivants :

- Contrôle positif : EGF (epidermal growth factor - facteur de croissance épidermique) à 10 ng/ml
- Spécialité commerciale Ascophyscient® d'Algues et Mer : 10 et 100 μg/ml (correspondant à 0.001 et 0,01 % respectivement)
- Hyaluronate de sodium 60- 120kDa selon l'exemple 1 : 100 et 1000 μg/ml (correspondant à 0,01 et 0,1 % respectivement)
- Association comprenant respectivement Ascophyscient® et hyaluronate de sodium 60-120 kDa aux concentrations respectives suivantes :

  - 10 μg/ml + 100 μg/ml,
  - 10 μg/ml + 1000 μg/ml.

Analyse des résultats :

- en DO (proportionnelle à l'incorporation de BrdU et donc au taux de prolifération cellulaire)
- en pourcentage de stimulation :

$$\left[ \frac{\textit{DO traité}}{\text{DO témoin}} \times 100 \right] - 100$$

- en erreur standard de la moyenne : esm = **écart-type (Sd)/√n**

Analyses statistiques :

Des analyses statistiques par le test T de Student bilatéral non apparié ont été réalisées sur les valeurs brutes de DO.

Ce test donne alors les valeurs des « p value » caractérisant la significativité des résultats obtenus pour les différentes conditions. Le degré de significativité est établi comme suit : significatif pour $p < 0,05$ (*)

très significatif pour $p < 0,01$ (**)
hautement significatif pour $p < 0.001$ (***)
non significatif pour $p > 0,05$

Résultats :

Dans ces conditions expérimentales,

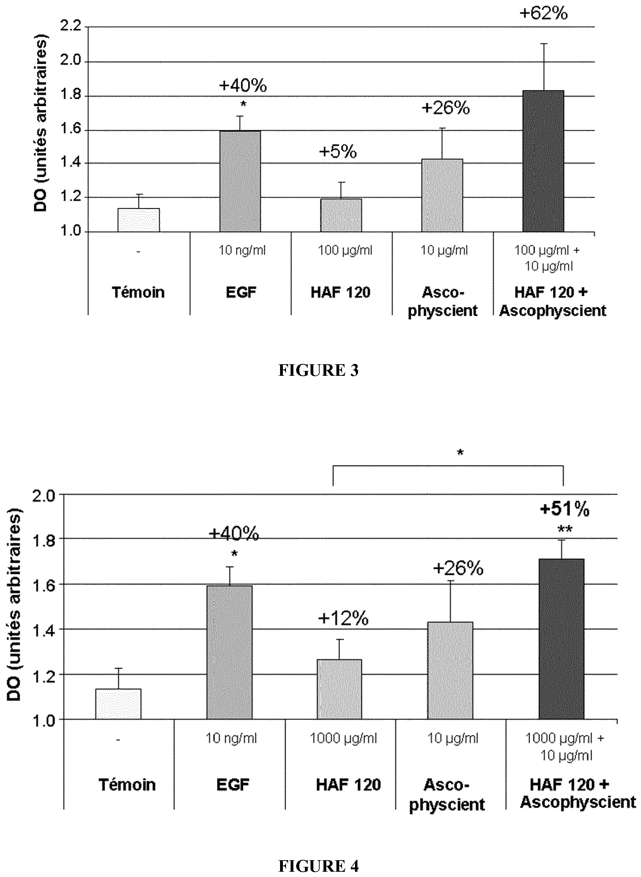

- Ascophyscient® a induit la prolifération des fibroblastes de façon très reproductible et statistiquement significative, surtout à 100 μg/ml (Figure 2).
- Le hyaluronate de sodium seul a un effet modéré sur la prolifération des fibroblastes (Figure 2).
- L'association de ces 2 composés a induit la prolifération des fibroblastes de façon synergique (Figures 3 et 4).

Conclusions :

Dans ces conditions expérimentales, Ascophyscient® a induit la prolifération des fibroblastes de façon très reproductible et statistiquement significative. L'association d'Ascophyscient® avec HAF120 a induit la prolifération des fibroblastes de façon synergique.

En induisant la prolifération des fibroblastes, Ascophyscient® et son association avec le fragment 60-120 kDa de hyaluronate de sodium restaure le métabolisme cellulaire et participe à la réparation dermique et à la lutte contre le vieillissement.

EXEMPLES DE COMPOSITION

*Exemple 1 :*

| Nom | Pourcentage |
|---|---|
| Eau purifiée | QSP 100 % |
| Glycérine | 6 |
| EDTA disodique | 0.1 |
| Phénoxyéthanol | 0.35 |
| Acrylates/C10-30 Alkyl Acrylate Crosspolymer (Carbopol® Ultrez 21) | 0.4 |
| Polyacrylate-13 & Polyisobutène & Polysorbate 20 & Eau (Sepiplus™ 400) | 1 |
| Glycéryl Stéarate & PEG-100 Stéarate (Simulsol™ 165) | 4 |
| Alcool Cétylique | 1 |
| Caprylic/Capric Triglycérides Capryliques/Capriques (Myritol® 318) | 10 |
| Diméthicone (DC 200) | 4 |
| Dicaprylyl Carbonate | 4 |
| Hyaluronate de sodium 60-120 kDa selon l'invention | 0.5 |
| Ascophyscient® | 0.3 |
| Parfum | 0.1 |

*Exemple 2 :*

| Nom | Pourcentage |
|---|---|
| Eau purifiée | QSP 100 % |
| Glycérine | 6 |
| EDTA disodique | 0.1 |
| Phénoxyéthanol | 0.35 |
| Acrylates/C10-30 Alkyl Acrylate Crosspolymer (Carbopol® Ultrez 21) | 0.4 |
| Polyacrylate-13 & Polyisobutène & Polysorbate 20 & Eau (Sepiplus™ 400) | 1 |
| Glyceryl Stéarate & PEG-100 Stéarate (Simulsol™ 165) | 4 |
| Alcool Cétylique | 1 |
| Triglycérides Capryliques/Capriques (Myritol® 318) | 10 |
| Diméthicone (DC 200) | 4 |
| Isododécane | 4 |
| Hyaluronate de sodium 60-120 kDa selon l'invention | 0.5 |
| Ascophyscient® | 0.3 |
| Rétinaldéhyde | 0.05 |
| Parfum | 0.1 |

**Revendications**

1. Association comprenant un acide hyaluronique ou un de ses sels dont la masse moléculaire moyenne en poids est comprise entre 60 et 120 kDa et un polysaccharide sulfaté dont le poids moléculaire est compris entre 5 et 25 kDa et qui est un fucane sulfaté.

2. Association selon la revendication 1, **caractérisée en ce que** le ratio massique acide hyaluronique / polysaccharide est compris entre 0,1 et 10.

3. Association selon la revendication 1 ou 2, pour son utilisation topique destinée à favoriser la prolifération des fibroblastes.

4. Utilisation topique d'une association selon la revendication 1 ou 2 pour lutter contre les signes du vieillissement cutané.

5. Association selon la revendication 1 ou 2, pour son utilisation topique destinée à traiter les lésions cutanées.

6. Association selon la revendication 1 ou 2, pour son utilisation topique destinée à améliorer la cicatrisation et la réparation cutanées.

7. Composition dermatologique ou cosmétique comprenant à titre de principe actif une association selon la revendication 1 ou 2, avec au moins un excipient dermatologiquement ou cosmétiquement acceptable.

8. Composition selon la revendication 7, **caractérisée en ce qu'**elle comprend en outre un autre principe actif.

9. Composition selon la revendication 8, **caractérisée en ce que** l'autre principe actif est choisi parmi des agents anti-âge, cicatrisants, apaisants, antiprurit, anti-radicalaires, anti-UV, stimulant la synthèse de macromolécules dermiques ou le métabolisme énergétique, hydratants, dépigmentants, antibactériens, antifongiques, anti-inflammatoires, anesthésiques et leurs mélanges.

10. Composition selon la revendication 8, **caractérisée en ce que** l'autre principe actif est du rétinaldéhyde.

11. Composition selon la revendication 8, **caractérisée en ce que** l'autre principe actif est du delta tocophéryl glucopyranoside.

12. Utilisation cosmétique de la composition cosmétique selon l'une quelconque des revendications 7 à 11 pour lutter contre les signes du vieillissement cutané.

13. Composition dermatologique selon la revendication 7, pour son utilisation dans le traitement des coupures, des sutures, des écorchures, des éraflures, des égratignures, des cicatrices post-chirurgie ou post-acte de dermatologie esthétique, des brûlures superficielles, des coups de soleil.


**Patentansprüche**

1. Verbindung, umfassend eine Hyaluronsäure oder ein Salz davon mit einem gewichtsmittleren Molekulargewicht zwischen 60 und 120 kDa und ein sulfatiertes Polysaccharid mit einem Molekulargewicht zwischen 5 und 25 kDa, und das ein sulfatiertes Fukan ist.

2. Verbindung nach Anspruch 1, **dadurch gekennzeichnet, dass** das Masseverhältnis Hyaluronsäure / Polysaccharid zwischen 0,1 und 10 liegt.

3. Verbindung nach Anspruch 1 oder 2, zur topische Anwendung zur Förderung der Proliferation der Fibroblasten.

4. Topische Verwendung einer Verbindung nach Anspruch 1 oder 2 zur Bekämpfung der Zeichen der Hautalterung.

5. Verbindung nach Anspruch 1 oder 2, zur topische Anwendung zur Behandlung von Hautverletzungen.

**6.** Verbindung nach Anspruch 1 oder 2, zur topische Anwendung zur Verbesserung der kutanen Heilung und Reparatur.

**7.** Dermatologische oder kosmetische Zusammensetzung, die als Wirkstoff eine Verbindung nach Anspruch 1 oder 2 mit zumindest einem dermatologisch oder kosmetisch akzeptablen Hilfsstoff umfasst.

**8.** Zusammensetzung nach Anspruch 7, **dadurch gekennzeichnet, dass** sie ferner einen anderen Wirkstoff umfasst.

**9.** Zusammensetzung nach Anspruch 8, **dadurch gekennzeichnet, dass** der andere Wirkstoff unter Anti-Aging-, Heilungs-, Beruhigungs-, Antipruritus-, Radikalfänger-, UV-Schutz-Stoffen, die die Synthese von dermalen Makromolekülen oder den Energiestoffwechsel anregen, Durchfeuchtungs-, Depigmentierungs-, antibakteriellen, antifungiellen, entzündungshemmenden, Anästhesiemitteln und Mischungen davon ausgewählt ist.

**10.** Zusammensetzung nach Anspruch 8, **dadurch gekennzeichnet, dass** der andere Wirkstoff Retinaldehyd ist.

**11.** Zusammensetzung nach Anspruch 8, **dadurch gekennzeichnet, dass** der andere Wirkstoff Delta-Tocopheryl-Glucopyranosid ist.

**12.** Kosmetische Verwendung der kosmetischen Zusammensetzung nach einem der Ansprüche 7 bis 11 zur Bekämpfung der Zeichen der Hautalterung.

**13.** Dermatologische Zusammensetzung nach Anspruch 7 zur Anwendung bei der Behandlung von Schnitten, Nähten, Schrammen, Kratzern, Abschürfungen, Narben nach Operationen oder ästhetischer Dermatologie, oberflächlichen Verbrennungen, Sonnenbränden.

**Claims**

**1.** Combination comprising a hyaluronic acid or a salt thereof having a weight average molecular weight between 60 and 120 kDa and a sulphated polysaccharide having a molecular weight between 5 and 25 kDa which is a sulphated fucan.

**2.** Combination according to claim 1, **characterized in that** the mass ratio of hyaluronic acid to polysaccharide is between 0.1 and 10.

**3.** Combination according to claim 1, for topical use intended to promote fibroblast proliferation.

**4.** Topical use of a combination according to claim 1 or 2, for combating the signs of skin aging.

**5.** Combination according to claim 1 or 2, for topical use intended to treat skin lesions.

**6.** Combination according to claim 1 or 2, for topical use intended to improve skin healing and repair.

**7.** Dermatological or cosmetic composition comprising as active ingredient a combination according to claim 1 or 2, with at least one dermatologically or cosmetically acceptable excipient.

**8.** Composition according to claim 7, **characterized in that** it further comprises another active ingredient.

**9.** Composition according to claim 8, **characterized in that** the other active ingredient is selected from anti-aging agents, healing agents, soothing agents, antipruritic agents, anti-radical agents, anti-UV agents, agents stimulating the synthesis of dermal macromolecules or energy metabolism, hydrating agents, depigmenting agents, antibacterials, antifungals, anti-inflammatories, anesthetics and mixtures thereof.

**10.** Composition according to claim 8, **characterized in that** the other active ingredient is retinaldehyde.

**11.** Composition according to claim 8, **characterized in that** the other active ingredient is delta-tocopheryl-glucopyranoside.

**12.** Cosmetic use of the cosmetic composition according to any one of claims 7 to 11 for combating the signs of skin aging.

**13.** Dermatological composition according to claim 7, for use in the treatment of cuts, sutures, abrasions, scratches, scrapes, scars following surgery or following an aesthetic dermatology procedure, superficial burns, sunburn.

**FIGURE 1**

**FIGURE 2**

FIGURE 3

FIGURE 4

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1987153 A

- WO 2010086197 A

**Littérature non-brevet citée dans la description**

- **TAMMI R. ; AGREN U.M. ; TUHKANEN A.L. ; TAMMI M.** Hyaluronan metabolism in skin. *Progress in Histochemistry & Cytochemistry,* 1994, vol. 29 (2), 1-81
- *Nat Rev Cancer,* 2004, vol. 4, 538-539

- **WITTE MB ; BARBUL A.** General principles of wound healing. *Surg Clin North Am.,* Juin 1997, vol. 77 (3), 509-28
- **STEPAN PODZIMEK.** *Solution of Hyaluronic Acid and Comparaison of SEC-MALLS-VIS Data with Off-line Capillary Viscometry Journal of Applied Polymer science,* 2009